Europäisches Patentamt

European Patent Office

Office européen des brevets

(19)

(11) Publication number: **0 181 976 B1**

## EUROPEAN PATENT SPECIFICATION

(12)

(45) Date of publication of patent specification: **11.09.91**

(21) Application number: **84307946.8**

(22) Date of filing: **15.11.84**

(51) Int. Cl.5: **A61B 17/32**, B26B 13/14, B26B 13/06

(54) **A cutting instrument.**

(43) Date of publication of application:
**28.05.86 Bulletin 86/22**

(45) Publication of the grant of the patent:
**11.09.91 Bulletin 91/37**

(84) Designated Contracting States:
**DE FR IT**

(56) References cited:
**DE-C- 402 695**       **GB-A- 942 775**
**US-A- 2 134 265**     **US-A- 2 865 099**
**US-A- 3 987 542**     **US-A- 4 133 107**
**US-A- 4 246 698**

(73) Proprietor: **LASCHAL SURGICAL, INC.**
**4 Baltusrol Drive**
**Purchase, New York 10577(US)**

(72) Inventor: **Lasner, Jeffrey**
**4 Baltusrol Drive**
**Purchase, New York 10577(US)**
Inventor: **Aleixo, Francisco**
**66 John Street**
**Tarrytown, New York 10591(US)**

(74) Representative: **Pendlebury, Anthony et al**
**PAGE, WHITE & FARRER 54 Doughty Street**
**London WC1N 2LS(GB)**

Rank Xerox (UK) Business Services

## Description

The present invention relates to cutting instruments such as an improved surgical suture remover.

From one aspect the invention provides an instrument that is capable of both cutting and gripping a suture so that a surgeon can, with one hand, cut the stitches and withdraw the cut thread, and that is readily manufacturable at relatively low cost with a high degree of precision and reliability.

In U.S 4,246,698 the applicants describe a suture remover surgical instrument of generally scissor-like configuration in which a pair of shanks are pivotally connected intermediate their ends for relative movement therebetween. The first shank carries on one of its ends a blade having a cutting section, while the second shank carries on its corresponding end a shearing portion having a cutting edge, so that the cutting section and cutting edge are movable toward and away from each other along a cutting plane. An elongated suture gripping element formed of a resilient material includes a contact area at one end and is attached as by soldering, at its other end to the shearing portion, the contact area being so disposed that as the cutting section and cutting edge are operatively moved toward each other along the cutting plane for shearing contact with a suture, the gripping element is deformed whereby its contact portion is resiliently moved along the cutting plane and substantially perpendicular to its elongation. In addition, a bend provided in and along the plane of the gripping element adjacent its end connection to the shearing portion advantageously facilitates distribution of the deformation of the gripping element between the bend and the end connection to prevent premature deterioration of the end connection and thereby extend the useful operational life of the instrument.

GB 942,775 describes disposable surgical scissors made from a single piece of resilient metal strip which is bent to form finger grips wich are parallel and terminate in blades which are pivotally connected together.

DE-C-402659 describes scissors made of flexible steel having two shanks spaced apart and co-operating cutting blades at one end. The blades are pivotally connected together and the shanks are twisted substantially 90° so that the blades are oriented in substantially overlapping planes.

It is a main object of the present invention to provide a cutting instrument which is an improvement over this prior teaching.

According to the invention there is provided a suture remover comprising an elongated strip of yieldable and resiliently flexible material which is bent to form two shanks which are spaced apart and have co-operating cutting blades at their remote ends, which blades are pivotally connected together, wherein both of the shanks are twisted substantially 90° in the same direction, whereby the blades are oriented in substantially overlying parallel planes and are capable of shearing action with approximately 0° clearance therebetween, wherein one of the blades has a cutting section, the other blade has a cutting edge, each of the shanks has an integral contact shoulder adjacent the twist for mutual abutment during suture cutting to prevent pivotal movement of said cutting section and cutting edge towards each other beyond a predetermined point and thereby prevent damage to the suture remover, and an elongated suture gripping element on the blade has a contact area and is integrally connected to the blade near the Pivot, which gripping element is substantially planar along its elongation and includes a bend connecting the gripping element to the blade so that the gripping element overlays a portion of the blade to which it is integrally connected so that relative movement of the cutting edge and cutting section for shearing contact with a suture causes deformation of the gripping element substantially perpendicular to the elongation of the gripping element as its contact area is resiliently moved along the cutting plane.

Some embodiments of the invention will now be described, by way of example, with reference to the accompanying drawings in which the same references indicate the same or similar parts, and in which:-

Figure 1 is a perspective view taken from below of an improved surgical suture remover in accordance with the invention,

Figure 2 is an enlarged elevated perspective view of the forward or suture cutting and gripping portion of the suture remover of Figure 1 taken from the top rear with respect to Figure 1,

Figure 3 is a rear face view with respect to Figure 1 of the forward portion of the suture remover of the invention,

Figure 4 is a front face view of the forward portion of the suture remover of Figure 1, illustrating the instrument in its normal, jaws open condition at the start of a suture cutting and gripping operation,

Figure 5 is a view similar to Figure 4 in which the operative forward portion of the instrument has been partially closed to grip the suture but prior to cutting thereof,

Figure 6 is a view similar to Figures 4 and 5 in which the forward portion of the instrument is shown subsequent to cutting of the suture wherein the jaws of the instrument are fully closed and the suture continues to be gripped to facilitate its withdrawal from the patient's tissue,

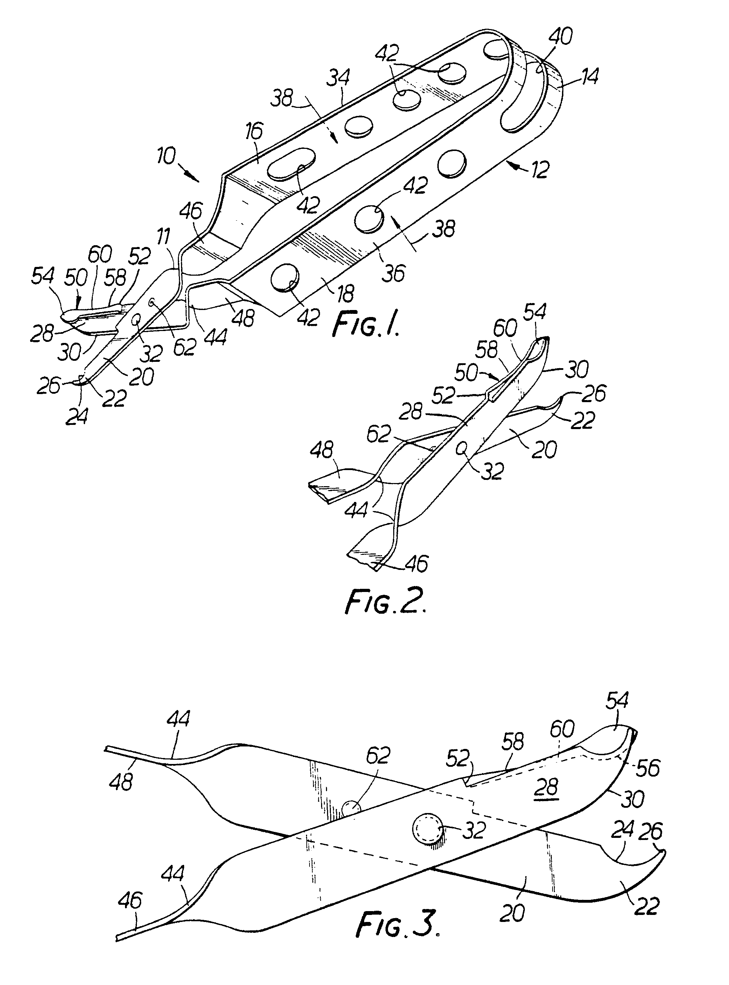

One embodiment of a suture remover in accordance with the invention, is a designated by the general reference numeral 10, as shown in Figure 1. The surgical instrument 10 is formed of a single, initially elongated strip 12 of a resiliently flexible and deformable material such as, by way of example only, stainless steel. Strip 12 is provided with a gradual, arcuate central bend 14 substantially midway in its elongation to define a first shank 16 and a second shank 18 disposed in substantially spaced apart relation.

First shank 16 unitarily carries, on its end remote from central bend 14, an integral blade 20 which includes a cutting section 22 proximate its free end. In the preferred embodiment herein disclosed, cutting section 22 is provided with an arcuately concave surface or portion 24 and terminates in an end or tip 26 that is preferably somewhat rounded or smoothed to prevent inadvertent tissue damage during operative use of the instrument 10 as will hereinafter become apparent.

Similarly, second shank 18 carries, on its end remote from central bend 14, an integral shearing portion 28 which includes a cutting edge 30.

A rivet 32 or other suitable means pivotally connects blade 20 and shearing portion 28 for relative movement of cutting section 22 and cutting edge 30 toward and away from each other along a cutting plane for shearing contact with a surgical suture to be cut. Movement of cutting section 22 and cutting edge 30 relatively toward each other is accomplished by the application of opposed forces to finger bearing surfaces or portions 34, 36 of first and second shanks 16, 18 respectively, these opposed forces being indicated and represented by the reference arrows 38 in Figure 1. Subsequent return movement of cutting section 22 and cutting edge 30 away from each other is effected by a resilient return urgency imparted by central bend 14 when the surgical user discontinues applying mutually opposed forces 38 to finger bearing surfaces 34, 36.

The strip of material 12 unitarily forming the surgical instrument 10 may advantageously be provided with cutouts of predetermined configuration and at predetermined locations along shanks 16, 18 and central bend 14 to thereby control the resilient properties and characteristics exhibited. In the preferred embodiment herein disclosed, central bend 14 includes an elongated slot 40 defined therein and extending along at least a portion of the arc of bend 14. Slot 40 has the effect of decreasing the magnitude of resilient return urgency imparted to suture remover 10 by the central bend with respect to that spring-type urgency which would otherwise be provided in the absence of such a cutout.

Similarly, one or a plurality of cutouts or apertures 42 may be predeterminately defined in first and second shanks 16, 18, and particularly in finger bearing surfaces 34, 36, to increase their flexibility. As a consequence of these cutouts 42, operative use of the instrument 10 as will hereinafter be described requires increased movement of shanks 16, 18 toward each other on application of opposed forces 38 thereto, thus correspondingly increasing the control provided a user of the instrument 10 in effecting a desired degree of relative movement between blade 20 and shearing portion 28. Those skilled in the art will of course recognize and understand that the precise number and size and configuration of the various cutouts 40, 42 to achieve a desired degree of flexibility and return urgency during operative use of suture remover 10 is at least partly dependent upon the characteristics of the chosen material of construction of resilient strip 12, although the illustrated form of elongated slot 40 in central bend 14 has been determined to be particularly advantageous in appropriately distributing the decrease in return urgency along the desired portion of the length of bend 14. It is, in any event, contemplated that forms of all of the cutouts 40, 42 other than those shown in the drawings might alternatively be employed.

The end of each of first and second shanks 16, 18 remote from central bend 14, at which the shanks are integrally and unitarily attached to blade 20 and shearing portion 28, respectively, incorporates a twist 44 of substantially $90°$ so as to dispose the blade and shearing portion substantially parallel to each other and generally perpendicular to finger bearing surfaces 34, 36 of first and second shanks 16, 18. Moving next along shanks 16, 18 in the direction of central bend 14, respective contact or abutment shoulders 46, 48 extend from twists 44 and cooperatively interact in the nature of a stop. As the application of opposed finger-induced forces 38 on shanks 16, 18 cause relative movement of the shanks toward each other during operative use of instrument 10, shoulders 46, 48 are correspondingly moved together until, at the end of an operating stroke or suture cutting and gripping closure, the shoulders abuttingly meet or contact. This mutual abutment prevents further pivotal movement of cutting section 22 and cutting edge 30 together along the cutting plane irrespective of the continued application of opposed forces 38 to finger bearing surfaces 34, 36. Consequently, damage to the suture remover 10 due to overclosure or like operative abuse is positively prevented by mutual abutment of shoulders 46, 48 at the predetermined point constituting the end of the permissable operating stroke of the instrument.

A suture gripping element 50 of generally elongated form integrally depends and is unitarily connected to shearing portion 28 at a bend 52. As perhaps best seen in Figure 2, gripping element 50, as well as the main body of shearing portion 28, is substantially planar and in the preferred form of instrument 10 gripping element 50 closely overlies a surface area of shearing portion 28 so that they lie in parallel and adjacent planes. It will accordingly be understood that bend 52 bridgingly extends between said adjacently parallel planes to unitarily connect gripping element 50 with shearing portion 28. Put another way, bend 52 displaces gripping element 50 from the plane of shearing portion 28 to an adjacently parallel and overlying position readily apparent in Figure 2.

The end of suture gripping element 50 remote from its connecting bend 52 is provided with a contact area 54. In the disclosed embodiment shown in the drawings, contact area 54 includes an arcuately convex surface or portion 56 for suture-gripping complimentary mating contact or engagement with the correspondingly concave surface 24 of cutting section 22 during operative use of the instrument 10. In a preferred form of the invention, contact area 54, and more particularly surface 56 thereof, extends beyond cutting edge 30 so that during an operating stroke suture remover 10 initially grips, and then cuts, the suture. An elongated arm 58 extends between contact area 54 and connecting bend 52 of gripping element 50, and is intermediately provided with a narrowed portion 60. This widthwise or transverse narrowing of arm 58 in the area 60 has the effect of distributing operative deformation of gripping element 50 predeterminately along its elongation during use of the instrument 10. More particularly, this resilient deformation of gripping element 50 is caused, as a consequence of the presence of narrowed portion 60, to occur primarily at the narrowed portion rather than at the integral connecting bend 52 where such deformation would otherwise primarily take place and, as a consequence, deterioration in the integrity of connecting bend 52 is minimized and the useful operative life of suture remover 10 correspondingly extended.

Blade 20 is further provided with a protruding dimple 62 or the like extending from its surface adjacently opposing shearing portion 28 for contact with the shearing portion face during a suture cutting and gripping stroke. As cutting section 22 and cutting edge 30 are operatively moved together and dimple 62 on blade 20 slides against the surface of shearing portion 28 rearwardly of rivet 32, the blade and shearing portion are moved slightly apart and, as a consequence, the forward or jaw portions of the blade and shearing portion, and more particularly cutting section 22 and cutting edge 30, are correspondingly urged together into assured suture shearing contact in the cutting plane during their movement toward each other. Dimple 62 additionally decreases friction between blade 20 and shearing portion 28 and thus facilitates operative suture cutting and gripping closure of the instrument 10. It should be clear that dimple 62 could alternatively be provided on the opposed or contact face of shearing portion 28, or the same or similar structures could be defined on both blade 20 and shearing portion 28 for cooperative engagement during an operating stroke of the instrument 10.

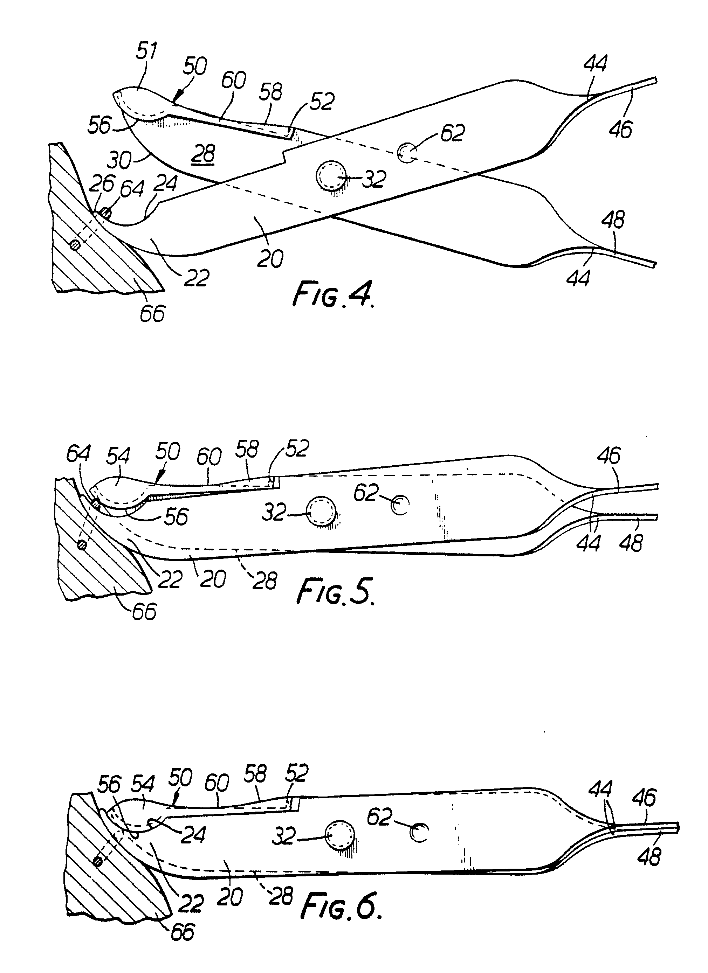

In use, and referring now to Figures 4 to 6, digging end 26 of blade 20 is inserted between a suture 64 and the tissue 66 in which the suture is tied. Opposed finger-induced forces 38 are applied to the bearing surfaces 34, 36 of shanks 16, 18 to effect an operating stoke or jaw closure of the instrument 10. As cutting section 22 and cutting edge 30 are thereby moved together, suture 64 is initially gripped or held between contact area 54 of gripping arm 50 and cutting section 22 of blade 20, or, more particularly, between complimentary surfaces 24 and 56, prior to actual severing of the suture thread (Figure 5). Further movement of cutting section 22 and shearing portion 28 together causes cutting edge 30, in cooperation with cutting section 22, to cut suture 64 while the grip of the suture between contact area 54 and cutting section 22 is maintained.

It should be noted that following initial gripping of the suture between contact area 54 and cutting section 22, the further relative movement of cutting section 22 toward cutting edge 30 along the cutting plane for shearing contact with the suture causes contact area 54 of gripping element 50 to be resiliently moved or forced along the cutting plane by reason of its continued contact with cutting section 22. In order to enable such continued movement of contact area 54, gripping element 50 is resiliently deformed substantially perpendicular to its elongation. This deformation is primarily concentrated in the arm 58 of gripping element 50 and, more particularly, at its narrowed portion 60. By so concentrating this resilient deformation at a somewhat central portion of gripping element 58, concentration of these deforming forces at connecting bend 52, which could otherwise severely stress the material at bend 52 causing premature material failure at that point is prevented, and the useful operative life of the suture remover is notably increased.

The length of the operating stroke or jaw closure of the instrument 10 is limited by the mutual abutment of contact shoulders 46, 48 as seen in Figure 6. In the absence of such a stroke limiting feature, the continued application of opposed forces 38 to shanks 16, 18 could easily result in the overstressing and consequent material failure of gripping element 50 as deformation thereof continues beyond its ability to resiliently flex.

The present invention accordingly teaches a surgical suture removal instrument advantageously formed of a single piece of material. With the exception of rivet 32, each and every element and structural feature of the disclosed preferred embodiment is inherently unitary and integral with every other element. As a consequence, there are no soldered or welded or other nonintegral connections to fail or at which unusual stresses on the instrument are likely to he concentrated, and the suture remover's reliability is correspondingly increased. Moreover, the relatively small number of requisite manufacturing steps enable this precision instrument to be manufactured at an extremely low production cost. Still further, the various structural features and elements of the one-piece suture remover cooperatively interact with one another to produce an instrument having operating characteristics and abilities beyond the mere sum of its collected parts.

## Claims

1. A suture remover comprising an elongated strip (12) of yieldable and resiliently flexible material which is bent to form two shanks (16, 18) which are spaced apart and have co-operating cutting blades (20, 28; 68, 78) at their remote ends, which blades are pivotally connected together, wherein both of the shanks (16, 18) are twisted (44) substantially 90° in the same direction, whereby the blades (20, 28; 68, 78) are oriented in substantially overlying parallel planes and are capable of shearing action with approximately 0° clearance therebetween, wherein one of the blades (20) has a cutting section (22), the other blade (28) has a cutting edge (30), each of the shanks (16, 18) has an integral contact shoulder (46, 48) adjacent the twist (44) for mutual abutment during suture cutting to prevent pivotal movement of said cutting section (22) and cutting edge (30) towards each other beyond a predetermined point and thereby prevent damage to the suture remover, and an elongated suture gripping element (50) on the blade (28) has a contact area (54) and is integrally connected to the blade (28) near the pivot (32), which gripping element (50) is substantially planar along its elongation and includes a bend (52) connecting the gripping element (50) to the blade (28) so that the gripping element (50) overlays a portion of the blade (28) to which it is integrally connected so that relative movement of the cutting edge (30) and cutting section (22) for shearing contact with a suture causes deformation of the gripping element substantially perpendicular to the elongation of the gripping element (50) as its contact area (54) is resiliently moved along the cutting plane.

2. A suture remover as claimed in Claim 1, characterised in that the cutting section (22) includes an arcuately concave portion (24) adjacent its end remote from the pivot (32), and the contact area (54) includes a correspondingly arcuately convex surface (56) for mating engagement with the concave portion (24) so as to enable a suture to be gripped therebetween as the cutting section (22) and the cutting edge (30) are moved towards each other along the cutting plane.

3. A suture remover as claimed in Claim 1 or Claim 2, characterised by a dimple (62) in at least one of the blades between the pivot (32) and the twist (44), which dimple is disposed in opposition to the other blade for contact therewith during operative use of the suture remover so as to urge said cutting section (22) and the cutting edge (30) into assured suture shearing contact during their movement towards each other.

4. A suture remover according to any one of Claims 1 to 3, characterised in that the gripping element (50) includes an elongated arm (58) integrally connecting the contact area (54) and the bend (52), which arm (58) has a narrowed portion (60) intermediate its ends for permitting the deformation of the gripping element (50) as the contact area (54) is resiliently moved along the cutting plane to occur primarily at the narrowed portion (60) rather than at the bend (52), thereby minimizing deterioration of the gripping element connection in use.

5. A suture remover as claimed in any one of Claims 1 to 4, characterised in that at least a portion of the contact area (54) of the gripping element (50) overlays and extends beyond the cutting edge (30) of the blade (28) so that movement of the cutting section (22) and cutting edge (30) towards each other causes the gripping element (50) initially to grip the suture prior to shearing contact with the suture.

6. A suture remover as claimed in any one of Claims 1 to 5, characterised in that the elongated strip (12) of resiliently flexible material has a cutout (40) at its bend (14) to decrease resilient return urgency and facilitate ready selective movement of the shanks (16, 18) toward each other during use.

## Revendications

1. Instrument pour enlever les fils de suture comprenant une bande allongée (12) de matériau souple et élastiquement flexible qui est courbée pour former deux branches (16, 18) qui sont espacées et comportent des lames coupantes (20,28; 68,78) inopérantes à leurs extrémités éloignées, lesquelles lames sont reliées de manière à pivoter, dans lequel les deux branches (16, 18) sont tordues (44) à substantiellement 90° dans la même direction, de manière que les lames (20, 28; 68, 78) soient orientées dans des plans parallèles se recouvrant substantiellement et soient capables d'une action de cisaillement avec un jeu d'environ 0° entre elles, dans lequel une des lames (20) comporte une section coupante (22), l'autre lame (28) comporte un bord coupant (30), chacune des branches (16, 18) comporte un épaulement de contact en partie intégrante (46, 48) adjacent à la torsion (44) pour buter l'un contre l'autre lors de la coupe du fil de suture afin d'empêcher le pivotement de ladite section de coupe (22) et dudit bord coupant (30) l'un vers l'autre au-delà d'un point prédéterminé et donc empêcher l'endommagement de l'instrument d'enlèvement de fils de suture, et un élément allongé de saisie de fil de suture (50) sur la lame (28) comporte une zone de contact (54) et est relié en partie intégrante à la lame (28) près du pivot (32), lequel élément de saisie (50) est substantiellement plat sur sa longueur et comprend un coude (52) reliant l'élément de saisie (50) à la lame (28) de sorte que l'élément de saisie (50) recouvre une portion de la lame (28) à laquelle il est relié en partie intégrante afin que le déplacement l'un par rapport à l'autre du bord coupant (30) et de la section coupante (22) pour le contact de cisaillement avec un fil de suture entraîne la déformation de l'élément de saisie substantiellement perpendiculairement à la longueur de l'élément de saisie (50) alors que sa zone de contact (54) est déplacée élastiquement le long du plan de coupe.

2. Instrument pour enlever les fils de suture selon la revendication 1, caractérisé en ce que la section coupante (22) comprend une portion concave arquée (24) adjacente à son extrémité éloignée du pivot (32), et la zone de contact (54) comprend une surface convexe arquée correspondante (56) pour un engagement complémentaire avec la portion concave (24) afin de permettre à un fil de suture d'être saisi entre elles alors que la section coupante (22) et le bord coupant (30) sont déplacés l'un vers l'autre le long du plan de coupe.

3. Instrument pour enlever les fils de suture selon la revendication 1 ou 2, caractérisé par une dépression (62) dans au moins une des lames entre le pivot (32) et la torsion (44), laquelle dépression est disposée en face de l'autre lame pour contacter celle-ci lors de l'actionnement de l'instrument d'enlèvement des fils de suture afin de forcer ladite section coupante (22) et ledit bord coupant (30) en un contact de cisaillement de fil de suture sûr lors de leur déplacement l'un vers l'autre.

4. Instrument pour enlever les fils de suture selon l'une quelconque des revendications 1 à 3, caractérisé en ce que l'élément de saisie (50) comprend un bras allongé (58) reliant en partie intégrante la zone de contact (54) et le coude (52), lequel bras (58) comporte une portion rétrécie (60) au milieu de ses extrémités pour permettre à la déformation de l'élément de saisie (50) alors que la zone de contact (54) est déplacée élastiquement le long du plan de coupe de se produire principalement à la portion rétrécie (60) plutôt qu'au coude (52), de manière à minimiser la détérioration de la connexion de l'élément de saisie à l'utilisation.

5. Instrument pour enlever les fils de suture selon l'une quelconque des revendications 1 à 4, caractérisé en ce qu'au moins une portion de la zone de contact (54) de l'élément de saisie (50) recouvre et s'étend au-delà du bord coupant (30) de la lame (28) de manière que le déplacement de la section coupante (22) et du bord coupant (30) l'un vers l'autre amène l'élément de saisie (50) à saisir initialement le fil de suture avant le contact de cisaillement avec le fil de suture.

6. Instrument pour enlever les fils de suture selon l'une quelconque des revendications 1 à 5, caractérisé en ce que la bande allongée (12) de matériau élastique comporte une découpe (40) à sa courbure (14) pour diminuer la force de rappel élastique et faciliter le déplacement sélectif immédiat des branches (16, 18) l'une vers l'autre durant l'utilisation.

**Patentansprüche**

1. Nahtentferner, bestehend aus einem länglichen Streifen (12) eines nachgebenden und federelastischen Materials, das gebogen ist, um zwei Schenkel (16, 18) zu bilden, welche im Abstand voneinander verlaufen und an ihren entfernten Enden zusammenwirkende Schneidklingen (20, 28; 68, 78) aufweisen, die schwenkbar miteinander verbunden sind, wobei die beiden Schenkel (16, 18) im wesentlichen in der gleichen Richtung um 90° verdreht (44) sind, wodurch die Klingen (20, 28; 68, 78) in im wesentlichen parallel übereinanderliegenden Ebenen ausgerichtet sind und eine Scherwirkung mit einem Zwischenraum von ca. 0° ausüben können, wobei die eine der Klingen (20) ein Schneidprofil (22), die andere Klinge (28) eine Schneidkante (30) aufweist, jeder der beiden Schenkel (16, 18) eine angeformte Kontaktschulter (46, 48) anschließend an die Verdrehung (44) als gegenseitigen Anschlag während des Naht-Schneidvorgangs zum Verhindern einer Schwenkbewegung des Schneidprofils (22) und der Schneidkante (30) gegeneinander über einen vorbestimmten Punkt hinaus enthält, um eine Beschädigung des Nahtentferners zu vermeiden, und ein länglicher Naht-Greifteil (50) an der Klinge (28) einen Kontaktbereich (54) enthält und einstückig mit der Klinge (28) in der Nähe des Drehpunkts (32) verbunden ist und der Greifteil (50) über seine Länge im wesentlichen eben ist und eine Kurve (52) umfaßt, welche den Greifteil (50) mit der Klinge (28) verbindet, so daß der Greifteil (50) einen Teil der Klinge (28) überdeckt, mit der er einstückig verbunden ist, so daß eine Relativbewegung der Schneidkante (30) und des Schneidprofils (22) bei scherender Berührung mit einer Naht eine Verformung des Greifteils im wesentlichen senkrecht zur Längsrichtung des Greifteils (50) bewirkt, wenn sein Kontaktbereich (54) federnd längs der Schnittebene bewegt wird.

2. Nahtentferner nach Anspruch 1, dadurch gekennzeichnet, daß das Schneidprofil (22) einen bogenförmig konkaven Teil (24) in der Nähe seines vom Drehpunkt (32) entfernten Endes enthält, und daß der Kontaktbereich (54) eine entsprechend bogenförmig konvexe Fläche (56) zur angepaßten Berührung mit dem konkaven Teil (24) enthält, um das Ergreifen einer Naht zwischen denselben zu ermöglichen, wenn das Schneidprofil (22) und die Schneidkante (30) längs der Schnittebene gegeneinander bewegt werden.

3. Nahtentferner nach Anspruch 1 oder 2, gekennzeichnet durch eine Erhebung (62) an mindestens einer der Klingen zwischen dem Drehpunkt (32) und der Verdrehung (44), wobei die Erhebung gegen die andere Klinge gerichtet ist, zur Berührung mit derselben während der funktionsgemäßen Benützung des Nahtentferners, um das Schneidprofil (22) und die Schneidkante (30) bei ihrer Bewegung gegeneinander in einen das Abscheren der Naht sicherstellenden Kontakt zu zwingen.

4. Nahtentferner nach einem der Ansprüche 1 bis 3, dadurch gekennzeichnet, daß der Greifteil (50) einen länglichen Arm (58) umfaßt, der den Kontaktbereich (54) einstückig mit der Kurve (52) verbindet, wobei der Arm (58) einen verengten Teil (60) zwischen seinen Enden aufweist, der die Verformung des Greifteils (50) ermöglicht, wenn der Kontaktbereich (54) elastisch längs der Schnittebene bewegt wird, und die Verformung vorwiegend eher an dem verengten Teil (60) als an der Kurve (52) erfolgt, wodurch die Abnützung der Verbindung des Greifteils bei der Benützung auf ein Minimum reduziert wird.

5. Nahtentferner nach einem der Ansprüche 1 bis 4, dadurch gekennzeichnet, daß mindestens ein Teil des Kontaktbereichs (54) des Greifteils (50) die Schneidkante (30) der Klinge (28) überdeckt und über dieselbe hinausragt, so daß eine Bewegung des Schneidprofils (22) und der Schneidkante (30) in Richtung zueinander bewirkt, daß der Greifteil (50) zunächst die Naht ergreift, bevor die Scherberührung mit der Naht erfolgt.

6. Nahtentferner nach einem der Ansprüche 1 bis 5, dadurch gekennzeichnet, daß der längliche Streifen (12) aus federelastischem Material eine Aussparung (40) an seiner Krümmung (14) aufweist, um die federnde Rückholkraft zu verringern und die gezielt selektive Bewegung der Schenkel (16, 18) in Richtung zueinander bei der Benützung zu erleichtern.

EP 0 181 976 B1

FIG.1.

FIG.2.

FIG.3.

8

FIG.4.

FIG.5.

FIG.6.